(19) Europäisches Patentamt
European Patent Office
Office européen des brevets

(11) Veröffentlichungsnummer: **0 190 233 B1**

(12) # EUROPÄISCHE PATENTSCHRIFT

(45) Veröffentlichungstag der Patentschrift: **09.12.92**

(51) Int. Cl.$^5$: **C07C 405/00**, A61K 31/557

(21) Anmeldenummer: **85903810.1**

(22) Anmeldetag: **18.07.85**

(86) Internationale Anmeldenummer:
**PCT/DE85/00246**

(87) Internationale Veröffentlichungsnummer:
**WO 86/00895 (13.02.86 86/04)**

Die Akte enthält technische Angaben, die nach dem Eingang der Anmeldung eingereicht wurden und die nicht in dieser Patentschrift enthalten sind.

(54) **NEUE CARBACYCLINE, VERFAHREN ZU IHRER HERSTELLUNG UND IHRE VERWENDUNG ALS ARZNEIMITTEL.**

(30) Priorität: **27.07.84 DE 3428266**

(43) Veröffentlichungstag der Anmeldung:
**13.08.86 Patentblatt 86/33**

(45) Bekanntmachung des Hinweises auf die Patenterteilung:
**09.12.92 Patentblatt 92/50**

(84) Benannte Vertragsstaaten:
**AT BE CH DE FR GB IT LI LU NL SE**

(56) Entgegenhaltungen:
**EP-A- 0 055 208**
**EP-A- 0 086 611**
**EP-A- 0 086 612**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **VORBRÜGGEN, Helmut**
**Wilkestr. 7**
**W-1000 Berlin 27(DE)**
Erfinder: **NIEUWEBOER, Bob**
**Carstennstr. 44**
**W-1000 Berlin 45(DE)**
Erfinder: **STÜRZEBECHER, Claus-Steffen**
**Brigittenstrasse 6a**
**W-1000 Berlin 46(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft neue Carbacyclin-Derivate, Verfahren zu ihrer Herstellung sowie ihre Verwendung als Arzneimittel. In der US-Patentschrift 4,420,632 werden 9-alkylierte Carbacyclin-Derivate beschrieben, die antithrombotische, antisekretorische und bronchiodilatierende Eigenschaften besitzen. Sie wirken außerdem thrombocytenaggregationshemmend. In EP 55208 werden 3-Oxa-carbacycline beschrieben, die in 9-Stellung nicht substituiert sind. Aus EP 86611 sind 9-substituierte Carbacycline bekannt, genauso wie aus EP 86612. In beiden Patentschiften werden jedoch keine längerkettigen Substituenten mit w-funktionellen Gruppen beschrieben. Es wurde gefunden, daß durch Substitution der Methylengruppe in 3-Position dieser Carbacycline durch Sauerstoff oder durch Kettenverlängerung in 9-Stellung biologisch aktive Derivate erhalten werden, die eine längere Wirkungsdauer, eine größere Selektivität und eine bessere Wirksamkeit besitzen. Die in 9-Stellung kettenverlängerten Derivate lassen sich bei nur geringem Verlust an biologischer Aktivität an polymere Träger binden. Die erfindungsgemäßen Verbindungen wirken bronchodilatorisch und sind zur Inhibierung der Thrombozytenaggregation, zur Blutdrucksenkung über eine Vasodilation und zur Hemmung der Magensäuresekretion geeignet.

Die Erfindung betrifft Carbacyclinderivate der allgemeinen Formel I

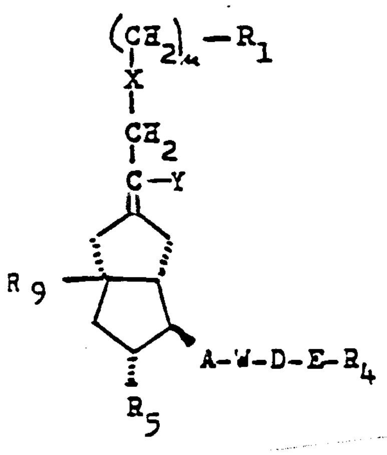

I,

worin

- n 1 oder 3
- $R_1$ den Rest $COOR_2$, wobei $R_2$ Wasserstoff darstellt,
- $R_9$ die Gruppe $-C{\equiv}C-(CH_2)_m-R_6$, worin
- m 1 bis 8 und
- $R_6$ Hydroxy oder Amino bedeutet,
- X ein Sauerstoffatom
- Y Wasserstoff
- A eine trans-CH = CH- oder -C≡C-Gruppe,
- W eine Hydroxymethylengruppe oder eine

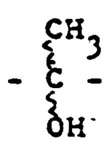

Gruppe, wobei die OH-Gruppe α- oder β-ständig sein kann,

- D eine geradkettige, gesättigte Alkylengruppe mit 1-5 C-Atomen oder eine verzweigte gesättigte Alkylengruppe mit 2-5 C-Atomen,
- E eine -C≡C-Gruppe,
- $R_4$ eine Alkylgruppe mit 1-4 C-Atomen,
- $R_5$ eine Hydroxygruppe, und, falls $R_2$ die Bedeutung eines Wasserstoffatoms hat, deren Salze mit physiologisch verträglichen Basen bedeuten.

Die Verbindungen der Formel I stellen sowohl (5E)- als auch (5Z)-Isomere dar.

Als Alkylgruppe $R_4$ kommen gerad- und verzweigtkettige, gesättigte und Alkylreste mit 1-4 C-Atomen,

in Frage.

Beispielsweise genannt seinen Methyl-, Äthyl-, Propyl-, Butyl-, Isobutyl-, tert.-Butyl-.

Als Alkylengruppe D kommen geradkettige oder verzweigtkettige, gesättigte Alkylenreste mit bis zu 5 C-Atomen, in Frage Beispielsweise seien genannt: Methylen, Äthylen, 1,2-Propylen, Äthyläthylen, Trimethylen, Tetramethylen, Pentamethylen, 1-Methyltetramethylen, 1-Methyl-trimethylen.

Besonders bevorzugte Verbindungen dieser Erfindung sind solche mit E als -C≡C-.

Unter den Resten -C≡C-$(CH_2)_m$-$R_6$ für $R_9$ sind Reste mit m = 1-8 bevorzugt.

Zur Salzbildung mit den freien Säuren ($R_2$ = H) sind anorganische und organische Basen geeignet, wie sie dem Fachmann zur Bildung physiologisch verträglicher Salze bekannt sind. Beispielsweise seien genannt: Alkalihydroxide, wie Natrium- und Kaliumhydroxid, Erdalkalihydroxide, wie Calciumhydroxid, Ammoniak, Amine, wie Äthanolamin, Diäthanolamin, Triäthaholamin, N-Methylglucamin, Morpholin, Tris-(hyroxymethyl)-methylamin usw.

Die Erfindung betrifft ferner ein Verfahren zur Herstellung der erfindungsgemäßen Carbacycline der allgemeinen Formel I, dadurch gekennzeichnet, daß man in an sich bekannter Weise eine Verbindung der allgemeinen Formel II,

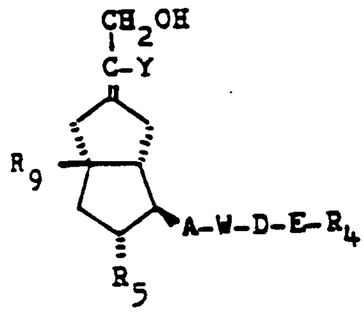

II

worin $R_4$, $R_5$, $R_9$, A, W, Y, D und E die obenangegebenen Bedeutungen aufweisen, gegebenenfalls nach Schutz anwesender freier Hydroxygruppen mit einem Halogenalkansäurederivat der allgemeinen Formel III,

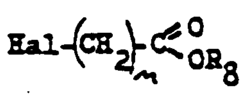

III

wobei n 1 oder 3, Hal ein Chlor- oder Bromatom und $R_8$ einen Alkylrest mit 1-4 C-Atomen oder ein Alkalimetall bedeutet, in Gegenwart einer Base veräthert und gegebenenfalls anschließend in beliebiger Reihenfolge Isomere trennt und/oder geschützte Hydroxygruppen freisetzt und/oder eine veresterte Carboxylgruppe verseift oder eine Carboxylgruppe mit einer physiologisch verträglichen Base in ein Salz überführt.

Die Umsetzung der Verbindung der allgemeinen Formel II mit einem Halogenalkansäurederivat der allgemeinen Formel III wird bei Temperaturen von 0°C bis 100°C, vorzugsweise 10° bis 80°C, in einem aprotischen Lösungsmittel oder Lösungsmittelgemisch, beispielsweise Dimethylsulfoxid, Dimethylformamid, Tetrahydrofuran usw., vorgenommen. Als Basen kommen die dem Fachmann für Verätherungen bekannten Basen in Frage, beispielsweise Natriumhydrid, Kalium-tert.-butylat, Butyllithium, usw.

Die Verseifung der Carbacyclinester wird nach den dem Fachmann bekannten Methoden durchgeführt, wie beispielsweise mit basischen Katalysatoren.

Die Carbacyclin-Derivate der allgemeinen Formel I mit $R_1$ in der Bedeutung einer Carboxygruppe können mit geeigneten Mengen der entsprechenden anorganischen Basen unter Neutralisierung in Salze überführt werden. Beispielsweise erhält man beim Lösen der entsprechenden PG-Säuren in Wasser, welches die stöchiometrische Menge der Base enthält, nach Abdampfen des Wassers oder nach Zugabe eines mit Wasser mischbaren Lösungsmittels,zum Beispiel Alkohol oder Aceton, das feste anorganische Salz.

Die Herstellung der Aminsalze erfolgt in üblicher Weise. Dazu wird die PG-Säure zum Beispiel in einem geeigneten Lösungsmittel, wie Äthanol, Aceton, Diäthyläther oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die Freisetzung einer funktionell abgewandelten OH-Gruppe zu den Verbindungen der allgemeinen Formel I erfolgt nach bekannten Methoden. Beispielsweise wird die Abspaltung von Ätherschutzgruppen in einer wäßrigen Lösung einer organischen Säure, wie zum Beispiel Essigsäure, Propionsäure u.a. oder in einer wäßrigen Lösung einer anorganischen Säure, wie zum Beispiel Salzsäure, durchgeführt. Zur Verbesserung der Löslichkeit wird zweckmäßigerweise ein mit Wasser mischbares inertes organisches Lösungsmittel zugesetzt. Geeignete organische Lösungsmittel sind zum Beispiel Alkohole, wie Methanol und Äthanol, und Äther, wie Dimethoxyäthan, Dioxan und Tetrahydrofuran. Tetrahydrofuran wird bevorzugt angewendet. Die Abspaltung wird vorzugsweise bei Temperaturen zwischen 20°C und 80°C durchgeführt.

Die Abspaltung der Silylätherschutzgruppen erfolgt beispielsweise mit Tetrabutylammoniumfluorid. Als Lösungsmittel sind beispielsweise geeignet Tetrahydrofuran, Diäthyläther, Dioxan, Methylenchlorid usw. Die Abspaltung wird vorzugsweise bei Temperaturen zwischen 0°C und 80°C durchgeführt.

Die Verseifung der Acylgruppen erfolgt beispielsweise mit Alkali- oder Erdalkali-carbonaten oder -hydroxyden in einem Alkohol oder der wäßrigen Lösung eines Alkohols. Als Alkohole kommen aliphatische Alkohole in Betracht, wie zum Beispiel Methanol, Äthanol, Butanol usw., vorzugsweise Methanol. Als Alkalicarbonate und -hydroxyde seien Kalium- und Natriumsalze genannt, bevorzugt sind jedoch die Kaliumsalze. Als Erdalkalicarbonate und -hydroxyde sind beispielsweise geeignet Calciumcarbonat, Calciumhydroxyd und Bariumcarbonat. Die Umsetzung erfolgt bei -10°C bis 70°C, vorzugsweise bei 25°C.

Enthält das Ausgangsprodukt OH-Gruppen im Prostanrest, so werden diese OH-Gruppen auch zur Reaktion gebracht. Werden letztlich Endprodukte gewünscht, die freie Hydroxylgruppen im Prostanrest enthalten, geht man zweckmäßigerweise von Ausgangsprodukten aus, in denen diese durch vorzugsweise leicht abspaltbare Äther- oder Acylreste intermediär geschützt sind.

Alle übrigen Verbindungen der Formel I können nach Verfahren hergestellt werden, wie sie in den Offenlegungsschriften DE-OS 28 45 770, 3237 200, 33 22 893 und 34 05 181 beschrieben werden. Wenn der Rest $R_9$ eine Alkinylgruppe darstellt, kann der Rest $R_9$ nach dem Verfahren von R.T. Hansen et.al. JACS, 100, 2244 (1978) eingeführt werden.

Die Verbindungen dieser Erfindung eignen sich insbesondere zur Therapie von Erkrankungen des cardiovaskulären Systems, des Magens, des Pankreas, der Leber und der Niere. Sie wirken blutdrucksenkend und bronchodilatorisch. Sie sind weiterhin geeignet zur Hemmung der Thrombozyten-Aggregation. Folglich stellen die neuen Carbacyclin-Derivate der Formel I wertvolle pharmazeutische Wirkstoffe dar. Darüber hinaus weisen sie bei ähnlichem Wirkungsspektrum, verglichen mit entsprechenden Prostaglandinen und Prostacyclinen, eine höhere Spezifität und vor allem eine wesentlich längere Wirksamkeit auf. Im Vergleich zu $PGI_2$ zeichnen sie sich durch größere Stabilität aus. Die hohe Gewebsspezifität der neuen Carbacycline zeigt sich bei der Untersuchung an glattmuskulären Organen, wie z.B. am Meerschweinchenileum oder an der isolierten Kaninchentrachea, wo eine wesentlich geringere Stimulation zu beobachten ist als bei der Applikation natürlicher Prostaglandine von E-, A- oder F-Typ.

Die neuen Carbacyclin-Analoga besitzen die für Prostacycline typischen Eigenschaften, wie z.B. Senkung des peripheren arteriellen und koronaren vaskülären Widerstandes, Inhibierung der Thrombozytenaggregation und Auflösung von Plättchenthromben, myocardiale Zytoprotektion, Senkung des systemischen Blutdruckes ohne zugleich Schlagvolumen und koronare Durchblutung zu senken; Behandlung von Schlaganfall, Prophylaxe und Therapie koronarer Herzerkrankungen, koronarer Thrombose, des Herzinfarkts, peripherer Arterienerkrankungen, Arteriosklerose und Thrombose, Prophylaxe und Therapie ischaemischer Attacken des ZNS-Systems, Therapie des Schocks, Inhibierung der Bronchokonstriktion, Inhibierung der Magensäuresekretion und Zytoprotektion der Magen- und Darmschleimhaut; Zytoprotektion in der Leber, im Pankcreas und in der Niere, antiallergische Eigenschaften, Senkung des pulmonalen vaskulären Widerstandes und des pulmonalen Blutdruckes, Förderung der Nierendurchblutung, Anwendung anstelle von Heparin oder als Adjuvans bei der Dialyse oder Hämofiltration, Konservierung von Blutplasmakonserven, besonders von Blutplättchenkonserven, Inhibierung von Gebutswehen, Behandlung von Schwangerschaftstoxikose, Erhöhung der zerebralen Durchblutung, Behandlung von Asthma, etc. Außerdem besitzen die neuen Carbacyclinanaloga antiproliferative Eigenschaften. Die neuen Carbacycline können außerdem in Kombination z.B. mit $\beta$-Blockern oder Diuretika Verwendung finden.

Die neuen Carbacycline zeichnen sich außerdem noch durch Unterdrückung von Abstoßungsreaktionen und durch ihre antimetastatische Wirkung aus. Durch sie wird der Ductus Botalli (vor Operationen) offengehalten. Sie eignen sich ferner zur Durchfallbehandlung und zur Verbesserung des Stuhlganges.

Die Carbacycline der Formel I, in denen $R_9$ den Rest $-C{\equiv}C-(CH_2)_m-R_6$ mit $R_6$ als OH- oder $NH_2$-Gruppe bedeutet, lassen sich sehr gut und ohne größeren Verlust an biologischer Aktivität an polymere Träger binden. Durch die neuen Carbacycline wird verhindert, daß sich an der Oberfläche dieser polymeren Träger wie z.B. Gefäßprothesen oder künstliche Herzklappen Thrombozytenaggregate bilden.

Die Dosis der Verbindungen ist 1-1500 $\mu$g/kg/Tag, wenn sie am menschlichen Patienten verabreicht

werden. Die Einheitsdosis für den pharmazeutischen akzeptablen Träger beträgt 0,01-100 mg.

Bei intravenöser Injektion an wachen, hypertonen Ratten in Dosen von 5, 20 und 100 µg/kg Körpergewicht zeigen die erfindungsgemäßen Verbindungen eine stärkere blutdrucksenkende und länger anhaltende Wirkung als-$PGE_2$ und $PGA_2$, ohne wie $PGE_2$ Durchfälle oder $PGA_2$ kardiale Arrhythmien auszulösen.

Bei intravenöser Injektion an narkotisierten Kaninchen zeigen die erfindungsgemäßen Verbindungen in Vergleich zu $PGE_2$ und $PGA_2$ eine stärkere und erheblich länger anhaltende Blutdrucksenkung, ohne daß andere glattmuskuläre Organe oder Organfunktionen beeinflußt werden.

Für die parenterale Verabreichung werden sterile, injizierbare, wässrige oder ölige Lösungen benutzt. Für die orale Applikation sind beispielsweise Tabletten, Dragees oder Kapseln geeignet.

Die Erfindung betrifft damit auch Arzneimittel auf Basis der Verbindungen der allgemeinen Formel I und üblicher Hilfs- und Trägerstoffe.

Die erfindungsgemäßen Wirkstoffe sollen in Verbindung mit den in der Galenik bekannten und üblichen Hilfsstoffen z.B. zur Herstellung von Blutdrucksenkern dienen.

Der Einheitsdosisbereich für die Ampulle ist 0,1 - 0,5 mg, für die Tablette 0,1 - 1 mg.

Beispiel 1

1β-Methyl-7α-(tetrahydropyran-2-yl-oxy)-6β-[3α-(tetrahydropyran-2-yl-oxy)-4-methyl-6,7-tetradehydro-trans-1-octenyl]-bicyclo[3.3.0]octan-3-on

Zu einer Suspension von 4,28 g (22,5 mmol) $Cu^{I}J$ in 25ml abs. Äther werden bei -5° vorsichtig unter Rühren 27 ml einer 1,6 N Methyllithiumlösung in Äther innerhalb von 15 Minuten zugetropft und zu dieser Lösung 2,21 g (5 mmol) 7α-(Tetrahydropyran-2-yloxy)-6β-[3α-(tetrahydropyran-2-yl-oxy)-4-methyl-6,7-tetradehydro-trans-1-octenyl]-bicyclo[3.3.0]oct-1-en-3-on (vgl. DE-OS 31 42 733) in 20 ml Äther bei -25° langsam während 15 Minuten zugetropft. Nach einer weiteren Stunde Rühren bei -25° bis -30° wird vorsichtig mit 100 ml $NH_4Cl$-Lösung versetzt und mit Äther extrahiert. Nach Waschen mit gesättigter Kochsalzlösung wird nach Trocknen und Abdampfen 2,37g Rohprodukt erhalten, das an einer Säule von 120g Silicagel chromatographiert wird. Elution mit Hexan-Äther (7:3) ergab 1,54 g (67%) des oben genannten Produktes.

Beispiel 2

3-Oxa-9β-methyl-16-methyl-18,19-tetradehydro-carbacyclin

Das unter Beispiel 1 beschriebene 5-Ring-Keton wird nach EP 55208 mit Dimethoxyphosphonoessigsäuremethylester in Gegenwart von Kalium-tert.-butylat umgesetzt, anschließend mit Lithiumaluminiumhydrid reduziert und der E-konfigurierte Alkylalkohol schließlich mit 2-Chlor- oder 2-Bromessigsäuresalzen oder-estern in Gegenwart von Basen mit nachfolgender Abspaltung der Tetrahydropyranylschutzgruppe zu 3-Oxa-9β-methyl-16-methyl-18,19-tetrahydrocarbacyclin umgesetzt.

## Patentansprüche

1. Carbacyclinderivate der allgemeinen Formel I

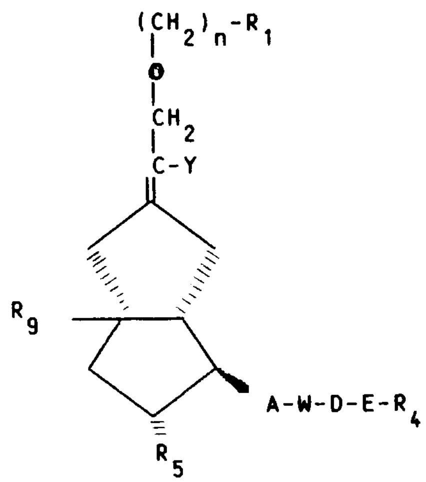

I,

worin

n 1 oder 3

$R_1$ den Rest $COOR_2$, wobei $R_2$ Wasserstoff darstellt,

$R_9$ die Gruppe $-C\equiv C(CH_2)_m-R_6$, worin

m 1 bis 8 und

$R_6$ Hydroxy oder Amino bedeutet,

Y Wasserstoff

A eine trans-CH = CH- oder -C≡C-Gruppe,

W eine Hydroxymethylengruppe oder eine

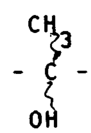

Gruppe, wobei die OH-Gruppe α- oder β-ständig sein kann,

D eine geradkettige, gesättigte Alkylengruppe mit 1-5 C-Atomen oder eine verzweigte gesättigte Alkylengruppe mit 2-5 C-Atomen,

E eine -C≡C-Gruppe,

$R_4$ eine Alkylgruppe mit 1-4 C-Atomen,

$R_5$ eine Hydroxygruppe, und falls $R_2$ die Bedeutung eines Wasserstoffatoms hat, deren Salze mit physiologisch verträglichen Basen bedeuten.

**2.** Verfahren zur Herstellung der Carbacycline der Formel I, dadurch gekennzeichnet, daß man in an sich bekannter Weise eine Verbindung der allgemeinen Formel II,

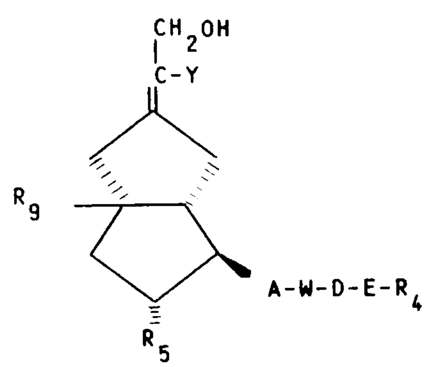

II,

worin $R_4$, $R_5$, $R_9$, A, W, Y, D und E die oben angegebenen Bedeutungen aufweisen, gegebenenfalls nach Schutz anwesender freier Hydroxygruppen mit einem Halogenalkansäurederivat der allgemeinen Formel III,

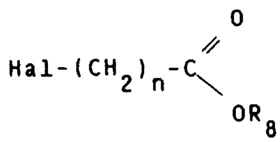

III

wobei n 1 oder 3, Hal ein Chlor- oder Bromatom und $R_8$ einen Alkylrest mit 1-4 C-Atomen oder ein Alkalimetall bedeutet, in Gegenwart einer Base veräthert und gegebenenfalls anschließend in beliebiger Reihenfolge Isomere trennt und/oder geschützte Hydroxygruppen freisetzt und/oder eine freie Carboxylgruppe verestert und/oder eine veresterte Carboxylgruppe verseift oder eine Carboxylgruppe mit einer physiologisch verträglichen Base in ein Salz überführt.

**3.** Arzneimittel, enthaltend eine oder mehrere Verbindungen gemäß Anspruch 1 und übliche Hilfs- und Trägerstoffe.

## Claims

**1.** Carbacyclin derivatives of the general formula I

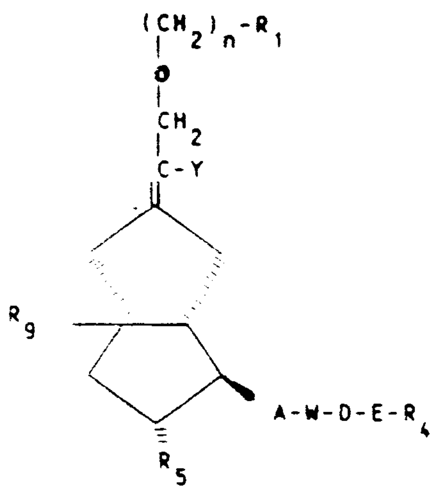

I,

wherein

| | |
|---|---|
| n | is 1 or 3, |
| $R_1$ | represents the radical $COOR_2$ wherein $R_2$ is hydrogen, |
| $R_9$ | represents the group $-C{\equiv}C(CH_2)_m-R_6$ wherein $\underline{m}$ is from 1 to 8 and $R_6$ represents hydroxy or amino, |
| Y | represents hydrogen, |
| A | represents a trans-CH = CH- or -C≡C- group, |
| W | represents a hydroxymethylene group or a |

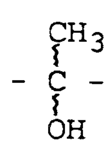

group wherein the OH group may be in the α- or β-configuration,

D represents a straight-chain, saturated alkylene group having from 1 to 5 carbon atoms, or a branched saturated alkylene group having from 2 to 5 carbon atoms,

E represents a -C≡C- group,

$R_4$ represents an alkyl group having from 1 to 4 carbon atoms, and

$R_5$ represents a hydroxy group, and, if $R_2$ represents a hydrogen atom, the salts thereof with physiologically tolerable bases.

**2.** Process for the preparation of the carbacyclins of formula I, characterised in that in a manner known per se, a compound of the general formula II

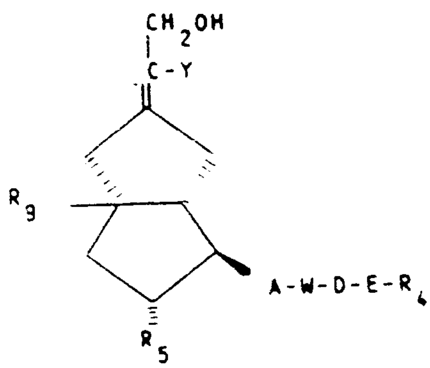

(II),

wherein $R_4$, $R_5$, $R_9$, A, W, Y, D and E have the meanings given above, optionally after the protection of free hydroxy groups present, is etherified, in the presence of a base, with a haloalkanoic acid derivative of the general formula III

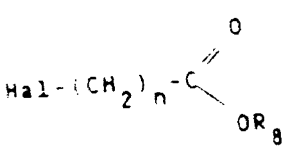

(III),

wherein n is 1 or 3, Hal represents a chlorine or bromine atom and $R_8$ represents an alkyl radical having from 1 to 4 carbon atoms or an alkali metal, and, where appropriate, then, in any sequence, isomers are separated and/or protected hydroxy groups are freed and/or a free carboxy group is esterified and/or an esterified carboxy group is hydrolysed or a carboxy group is converted into a salt with a physiologically tolerable base.

**3.** Medicament, comprising one or more compounds according to claim 1 and conventional excipients and carriers.

## Revendications

**1.** Carbacyclines répondant à la formule générale I :

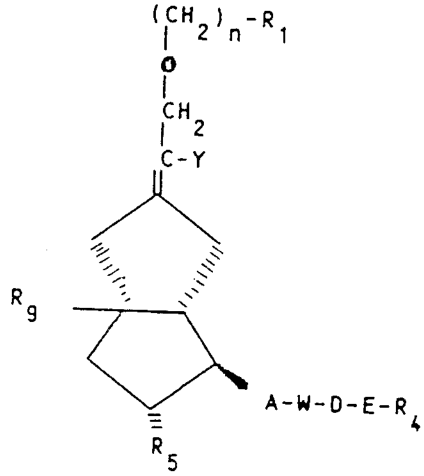

I,

dans laquelle

n est égal à 1 ou à 3,

$R_1$ représente un radical $-COOR_2$ dont le symbole $R_2$ désigne l'hydrogène,

$R_9$ représente un radical $-C{\equiv}C-(CH_2)_m-R_6$ dans lequel

m désigne un nombre de 1 à 8 et

$R_6$ représente un radical hydroxy ou un radical amino,

Y représente l'hydrogène,

A représente un radical -CH = CH- trans ou un radical $-C{\equiv}C-$,

W représente un radical hydroxyméthylène ou un radical

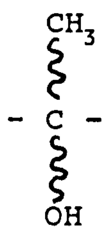

,

le radical -OH pouvant avoir la configuration $\alpha$ ou la configuration $\beta$ ,

D représente un radical alkylène linéaire saturé contenant de 1 à 5 atomes de carbone ou un radical alkylène ramifié saturé contenant de 2 à 5 atomes de carbone,

E représente un radical $-C{\equiv}C-$,

$R_4$ représente un radical alkyle contenant de 1 à 4 atomes de carbone et

$R_5$ représente un radical hydroxy,

ainsi que, lorsque $R_2$ représente un atome d'hydrogène, les sels que forment ces composés avec des bases acceptables du point de vue physiologique.

**2.** Procédé pour préparer les carbacyclines de formule I, procédé caractérisé en ce que, en opérant de manière connue, on éthérifie en présence d'une base un composé répondant à la formule générale II :

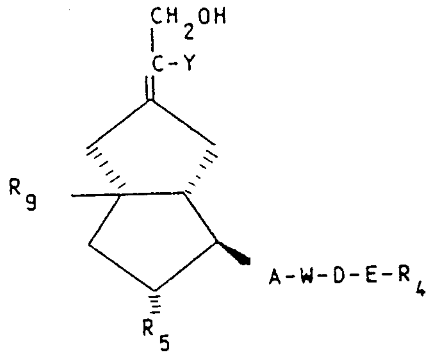

II,

dans laquelle $R_4$, $R_5$, $R_9$, A, W, Y, D et E ont les significations qui leur ont été données ci-dessus,

éventuellement après avoir protégé des radicaux hydroxy libres présents, avec un dérivé d'acide halogénoalcanoïque répondant à la formule générale III :

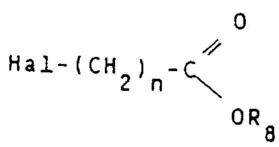

III

dans laquelle n est égal à 1 ou à 3, Hal représente un atome de chlore ou de brome et $R_8$ représente un radical alkyle contenant de 1 à 4 atomes de carbone ou un métal alcalin,

et ensuite, éventuellement, en opérant dans l'ordre que l'on veut, on sépare des isomères et/ou on libère des radicaux hydroxy protégés et/ou on estérifie un radical carboxy libre et/ou on saponifie un radical carboxy estérifié ou on transforme un radical carboxy en un sel avec une base acceptable du point de vue physiologique.

**3.** Médicament contenant un ou plusieurs composés selon la revendication 1 ainsi que des adjuvants et excipients usuels.